# EUROPEAN PATENT APPLICATION

(11) **EP 2 201 938 A1**
(43) Date of publication of application: **30.06.2010**
(21) Application number: 08172073.2
(22) Date of filing: 18.12.2008
(51) Int. Cl.: A61K 9/22

(54) **Controllable drug delivery capsule**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

An ingestible capsule (10) is provided for delivering a drug to an environment of the capsule (10), the capsule (10) comprising a compressible drug reservoir (13), an actuator, a pressure transferring element (15) and an electronic circuit (16). The compressible drug reservoir (13) comprises the drug and a delivery window (18) for upon compression of the drug reservoir (13) delivering the drug to the environment. The actuator compartment (12) comprises a swelling agent for swelling in response to a predefined environmental condition for the swelling agent, the swelling resulting in an increased pressure in the actuator compartment (12). The pressure transferring element (15) is provided for transferring the increased pressure in the actuator compartment to a compression of the drug reservoir (13). The electronic circuit (16) is configured to control when the delivering of the drug is performed.

## Description

### FIELD OF THE INVENTION

This invention relates to an ingestible capsule for delivering a drug to an environment of the capsule, the capsule comprising a compressible drug reservoir, an actuator and a pressure transferring element. The compressible drug reservoir comprises the drug and a delivery window for upon compression of the drug reservoir delivering the drug to the environment. The actuator compartment comprises a swelling agent for swelling in response to a predefined environmental condition for the swelling agent, the swelling resulting in an increased pressure in the actuator compartment. The pressure transferring element is provided for transferring the increased pressure in the actuator compartment to a compression of the drug reservoir.

### BACKGROUND OF THE INVENTION

Such an ingestible capsule is, e.g., known from WO 98/17260 A1, wherein a controlled drug delivery device is disclosed. The device includes a first compartment separated from a second compartment by a moveable partition. The first compartment includes a side having a screen or membrane. The second compartment includes an orifice. The first compartment contains a hydrogel and the second compartment contains a biologically active material or drug. The screen or membrane retains the hydrogel within the compartment but allows communication with fluids in a biological environment into which the device is placed. The hydrogel is selected to undergo a volume change such as an expansion in response to the occurrence of an environmental condition such as pH. Upon occurrence of such a condition, the hydrogel expands, thereby moving the moveable partition. This movement will decrease the volume in the second compartment, causing the biologically active material to exit through the orifice into a biological environment.

It is an advantage of such an ingestible capsule that it is somehow possible to select at what position inside the digestive system of the patient the drug delivery will start. Different parts of the digestive system have different pH values. By selecting a suitable hydrogel, which is responsive to a certain pH range, the place of delivery can be selected. However, pH values in the digestive system may show local and unexpected variations. Once the drug delivery starts, all drugs will be delivered within a short time span. For a more selective and targeted drug delivery, it would be desirable to control the drug delivery in a more effective way.

### OBJECT OF THE INVENTION

It is an object of the invention to provide a drug delivery device of which the drug delivery can be controlled in a more reliable way.

### SUMMARY OF THE INVENTION

According to a first aspect of the invention, this object is achieved by providing an ingestible capsule for delivering a drug to an environment of the capsule, the capsule comprising a compressible drug reservoir, an actuator, a pressure transferring element and an electronic circuit. The compressible drug reservoir comprises the drug and a delivery window for upon compression of the drug reservoir delivering the drug to the environment. The actuator compartment comprises a swelling agent for swelling in response to a predefined environmental condition for the swelling agent, the swelling resulting in an increased pressure in the actuator compartment. The pressure transferring element is provided for transferring the increased pressure in the actuator compartment to a compression of the drug reservoir. The electronic circuit is configured to control when the delivering of the drug is performed.

As will be elucidated below, the control of the moment of performing the drug delivery may be implemented in several ways. The control may involve, e.g., either the swelling, the transfer of pressure to the drug reservoir or the drug delivery itself. By, e.g., initiating, accelerating or slowing down one or more sub steps of the drug delivery process, the controllability of the location in the digestive system where the drug is delivered, is significantly improved. This improved controllability enhances the effectiveness of the drug. As a result, the required amount of drugs to be taken by the patient may be reduced, which may lead to a reduction of negative side effects.

In an embodiment of the ingestible capsule according to the invention, the electronic circuit is configured to control the environmental condition of the swelling agent. By controlling the environment of the swelling agent, also the moment at which the swelling will start is controlled. Also the rate of swelling depends on the environment of the swelling agent. The swelling agent may, e.g., swell in response to contact with a particular substance, in an environment at a particular temperature or in an environment with a particular pH level. The environmental conditions of the swelling agent may, e.g., be controlled by opening a valve which separates the swelling agent from the environment of the capsule, by heating the swelling agent or by actively releasing one or more particular substances. Similar measures may be taken for accelerating, slowing down or stopping the swelling of the swelling agent. In another embodiment of the ingestible capsule according to the invention, the electronic circuit is configured to control the pressure transferring element. The pressure transferring element may, e.g., be a piston which separates the swelling agent from the drug reservoir. As the swelling agent pushes against the piston, the piston compresses the drug reservoir and the drug is pushed out through the delivery window. When the electronic circuit is configured to control the pressure transferring element, the coupling between the swelling of the agent and the compression of the drug reservoir may selectively be activated and de-activated. Alternatively, a brake system for the piston may be controlled to adjust the speed of the drug delivery.

In another embodiment of the ingestible capsule according to the invention, the electronic circuit is configured to control opening of the delivery window. For example, the delivery window comprises an electronically controllable delivery valve and the electronic circuit is coupled to the delivery valve for selectively opening and closing the delivery window, the electronic circuit being coupled to the delivery valve for controlling the delivery valve. While the pressure in the actuator and the pressure exerted upon the drug reservoir increases, the delivery valve may still be closed. When the electronic circuit opens the delivery valve, the drug will be released very fast, because of the high pressure built up inside the capsule. The opening of the delivery valve may, e.g., be initiated in dependence of a received signal from an external communication device or in dependence of a signal from a sensor of the capsule. Such a sensor may, e.g., measure temperature, pH, time, presence of certain substances, etc.

These and other aspects of the invention are apparent from and will be elucidated with reference to the embodiments described hereinafter.

### BRIEF DESCRIPTION OF THE DRAWINGS

In the drawings:
Fig. 1 schematically shows an ingestible capsule according to the invention, and
Fig. 2 schematically shows three alternative embodiments of ingestible capsules according to the invention.

### DETAILED DESCRIPTION OF THE INVENTION

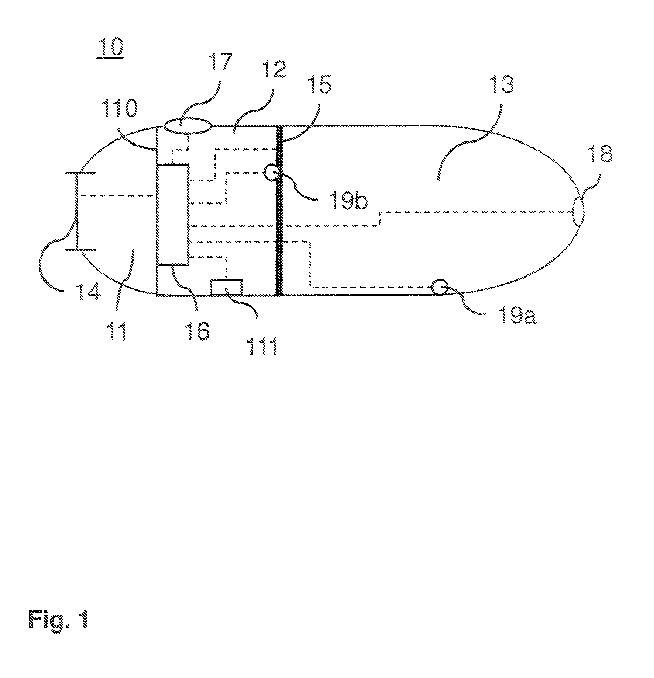

Fig. 1 schematically shows an ingestible capsule 10 according to the invention. The ingestible capsule 10 comprises an actuator compartment 12 and a compressible drug reservoir 13. The drug reservoir 13 comprises the drug to be delivered to the external environment of the capsule 10. The drug may, e.g., be stored in powder form, in microcapsules or dispensed in a liquid or a gel. The drug reservoir 13 comprises a delivery window 18 through which the drug is delivered to the environment. The opening of the delivery window 18 may, e.g., be controlled by an electronic circuit 16 for controlling the drug delivery process. The drug reservoir 13 in the capsule of Fig. 1 is compressible by moving a piston 15 in the direction of the drug reservoir 13 and the delivery window 18. The compression of the drug reservoir 13 results in an increased pressure inside the drug reservoir 13. A pressure sensor 19a inside the drug reservoir 13 may trigger the electronic circuit 16 to (temporarily) open the delivery window 18. Alternatively, the delivery window 18 may burst open when a critical amount of pressure is applied to it. In another embodiment, the delivery window 18 may comprise a membrane which melts when heated. Heating may be caused by an electrical current coming from the electronic circuit 16. Alternatively, electrical induction may be used to trigger melting of the membrane. This would enable a patient or doctor to initiate drug delivery by an ingested capsule 10 at any desired moment in time.

The actuator compartment 12 comprises a swelling agent for realizing the compression of the drug reservoir 13. When the swelling agent swells, the pressure inside the actuator compartment 12 is increased. When the pressure inside the actuator compartment 12 is higher than the pressure inside the drug reservoir 13, the piston 15 will move towards the drug reservoir 13 and the delivery window 18. This may result in delivery of the drug to the environment. Friction between the piston 15 and the capsule wall may slow down the movement of the piston 15. The friction between the piston 15 and the capsule wall may, e.g., be controlled by heating/cooling the piston 15 or capsule wall material. The heating/cooling may be controlled by the electronic circuit 16 or may be triggered from the outside via electrical induction.

Optionally, movement of the piston 15 is blocked and the blockade is removed upon a trigger from the electronic circuit 16. The electronic circuit 16 may, e.g., provide such a trigger when a pressure sensor 19b detects a predefined increase of pressure inside the actuator compartment. The trigger to remove the blockade may also depend on, e.g., a time or a temperature.

The swelling of the swelling agent in the actuator compartment 12 may occur in response to a change of environment. For example, a change of temperature or contact with a particular substance may cause the swelling of the swelling agent. The swelling agent may, e.g., be a pH-sensitive hydrogel which swells when it is in an environment within a certain pH-range. Also hydrogels are known which swell in response to contact with particular molecules. In the actuator compartment 12, a reservoir 111 may be provided for releasing such a special molecule which makes the swelling agent swell or for releasing substances influencing the pH level of the environment inside the actuator compartment 12. The reservoir is preferably coupled to the Instead of the reservoir 111, heating means may be provided for heating a temperature responsive swelling agent. The release of substance and the heating may be controlled by the electronic circuit 16.

Contact with particular molecules or with an environment at a predefined pH-level may also be realized by bringing the swelling agent into contact with body fluid of the patient. It is known that the composition of body fluid inside the digestive system of humans and animals strongly depends on the specific location in the digestive system. By selecting a suitable swelling agent, the position inside the digestive system, where the drug delivery will start can be chosen.

A body fluid compartment 11 may be provided for sampling and comprising body fluid from the external environment of the ingested capsule 10. The body fluid compartment 11 may come into contact with the swelling agent in the actuator compartment 12 via a semi-permeable membrane 110 or via a body fluid entrance in a wall of the actuator compartment 12. The body fluid entrance in the actuator compartment wall may be selectively opened by the electronic circuit 16 or may be a membrane which melts at a certain temperature, pH value or other environmental condition. Of course, melting of the membrane may again be initiated by the electronic circuit 16 or by electrical induction. In Fig. 1, the body fluid compartment 11 is separated from the external environment by a body fluid gate 14. Opening of this body fluid gate may again be, e.g., realized by the electronic circuit 16, electrical induction from an outside source or automatic opening of the gate in response to particular environmental conditions such as temperature or pH level.

Most swelling agents require water for changing its composition in a way resulting in swelling of the agent. This water may be obtained from the body fluid, but may also be provided by a reservoir 111 inside (or coupled to) the actuator compartment 12.

The electronic circuit 16 is situated somewhere in the capsule, preferably in or next to the actuator compartment 12. A communication module 17 may be provided for communicating sensor data to and/or receiving operation instructions from the user or the doctor. In a preferred embodiment, the capsule is a modular system with separate modules. A first module may comprise the actuator and the electronic circuit 16 and optionally some further features. A second module may comprise the drug reservoir 13. When using such a modular capsule 10, the production processes could be different for the different modules. For example, the drug reservoir module may be produced in a more sterile environment than the actuator/electronics module. Furthermore different drug reservoirs 13 with different drugs could be applied to the same actuator/electronics module.

Additionally, the capsule 10 may comprise one or more sensors. Such a sensor may, e.g., measure temperature, pH, time, presence of certain substances, etc. The sensors are coupled to the electronic circuit 16 for controlling the drug delivery based on the sensor measurements.

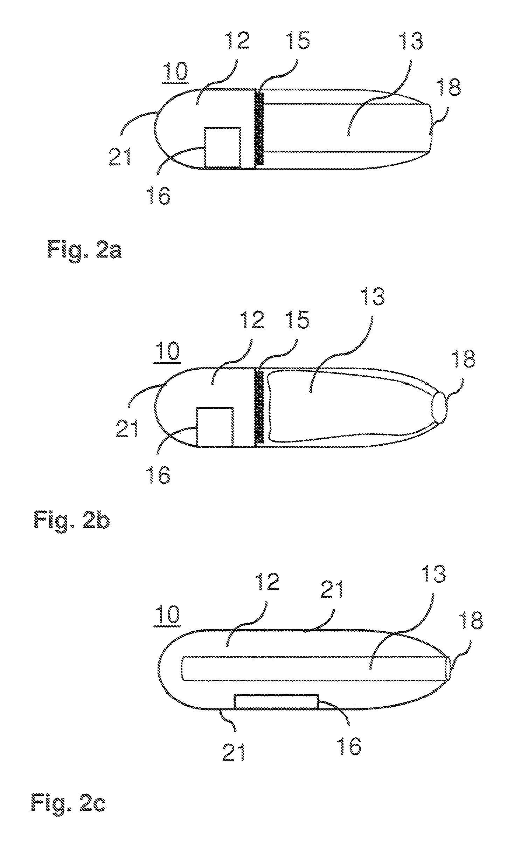

Figs. 2a, 2b and 2c schematically show three alternative embodiments of ingestible capsules 10 according to the invention. In Figs. 2a and 2b, a piston 15 is provided for transferring the increased pressure in the actuator compartment 12 to the drug reservoir 13. In Fig. 2a, the drug reservoir 13 comprises the drug in a solidified dry form. While the piston 15 is pushed towards the drug reservoir 13, the drug is pushed through the delivery window 18. Outside the delivery window 18, in the external environment of the capsule 10, the drug will dissolve in the body fluid of the patient.

In Fig. 2b, the piston 15 compresses a flexible envelope or balloon 13. The drug may be comprised in the balloon 13 in powder form. When pressure inside the balloon 13 increases, a membrane in the delivery window 18 bursts open or the delivery window is opened by the electronic circuit 16. The increased pressure inside the balloon 13 will then cause the powder to be ejected.

It is possible to provide a capsule 10 without a piston. For example, the swelling agent itself may push against the wall of the drug reservoir 13 to compress the reservoir. A membrane may be provided in between the swelling agent and the reservoir 13, which membrane bursts when the pressure from the actuator compartment 15 is above a critical value. As soon as this membrane bursts open, the swelling agent starts compressing the drug reservoir 13.

In Fig. 2c, the drug is suspended or dissolved in a liquid, gel or pasta. A large part of the drug reservoir 13 is surrounded by the swelling agent. The drug reservoir 13 has a flexible wall for separating the drug from the swelling agent. If the liquid/gel/pasta in the drug reservoir 13 do not mix with the swelling agent, the drug reservoir wall is not required. When the pressure in the actuator compartment 12 increases, the drug is squeezed out like in a toothpaste tube.

In the embodiments of Fig. 2, semi-permeable membranes 21 allow body fluid to enter the actuator compartment. All embodiments shown in Fig. 2 do comprise an electronic circuit 16 for controlling the drug delivery in some way. For example the drug delivery is controlled in any of the ways as described above with reference to Fig. 1. The electronic circuit 16 may, e.g., melt membranes, open gates or heat the swelling agent. Also the reservoir 111, the body fluid compartment 11, the communication means 17 and pressure sensors 19a, 19b may be used in the embodiments of Fig. 2.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. An ingestible capsule (10) for delivering a drug to an environment of the capsule (10), the capsule (10) comprising:
- a compressible drug reservoir (13) comprising the drug and a delivery window (18) for upon compression of the drug reservoir (13) delivering the drug to the environment,
- an actuator compartment (12) comprising a swelling agent for swelling in response to a predefined environmental condition for the swelling agent, the swelling resulting in an increased pressure in the actuator compartment (12),
- a pressure transferring element (15) for transferring the increased pressure in the actuator compartment (12) to a compression of the drug reservoir (13),
- an electronic circuit (16) being configured to control when the delivering of the drug is performed.

2. An ingestible capsule (10) as claimed in claim 1, wherein the electronic circuit (16) is configured to control the environmental condition of the swelling agent.

3. An ingestible capsule (10) as claimed in claim 2, wherein the actuator compartment (12) comprises an electronically controllable input valve, coupled to the electronic circuit (16) for selectively allowing fluidic contact of the swelling agent with the environment of the capsule (10) via the input valve.

4. An ingestible capsule (10) as claimed in claim 2, wherein the actuator compartment (12) comprises a water supply (111), coupled to the electronic circuit (16) for selectively allowing contact of the swelling agent with water from the water supply (111).

5. An ingestible capsule (10) as claimed in claim 1, wherein the electronic circuit (16) is configured to control the pressure transferring element (15).

6. An ingestible capsule (10) as claimed in claim 5, wherein the pressure transferring element is a piston (15), which piston (15) is arranged such that a pressure exerted on the piston (15) by the swelling agent results in the compression of the drug reservoir (13) by the piston (15).

7. An ingestible capsule (10) as claimed in claim 5, wherein the actuator compartment (12) comprises a pressure sensor (19b) for measuring a pressure inside the actuator compartment (12), the pressure sensor (19b) being coupled to the electronic circuit (16), the electronic circuit (16) being configured to control the pressure transferring element (15) in dependence of the measured pressure inside the actuator compartment (12).

8. An ingestible capsule (10) as claimed in claim 1, wherein the pressure transferring element (15) is a membrane, which membrane is arranged such that a pressure exerted on the membrane by the swelling agent results in opening of the membrane and in compression of the drug reservoir (13) by the swelling agent.

9. An ingestible capsule (10) as claimed in claim 1, wherein the delivery window (18) comprises an electronically controllable delivery valve for selectively opening and closing the delivery window (18), the electronic circuit (16) being coupled to the delivery valve for controlling the delivery valve.

10. An ingestible capsule (10) as claimed in any one of the preceding claims, further comprising at least one sensor for determining a condition in the environment of the capsule (10), the sensor being coupled to the electronic circuit (16), the electronic circuit (16) being configured to operate in dependence of the condition of the environment.
